(19) Europäisches Patentamt
European Patent Office
Office européen des brevets

(11) Veröffentlichungsnummer: **0 156 091**
**B1**

# (12) EUROPÄISCHE PATENTSCHRIFT

(45) Veröffentlichungstag der Patentschrift: **10.08.88**

(51) Int. Cl.⁴: **C 07 D 207/337**, A 61 K 31/40

(21) Anmeldenummer: **84810147.3**

(22) Anmeldetag: **27.03.84**

(54) **Beta-Oxo-alpha-carbamoyl-pyrrolpropionitrile, Verfahren zu ihrer Herstellung, pharmazeutische Präparate enthaltend diese Verbindungen, sowie ihre therapeutische Verwendung.**

(43) Veröffentlichungstag der Anmeldung:
**02.10.85 Patentblatt 85/40**

(45) Bekanntmachung des Hinweises auf die Patenterteilung:
**10.08.88 Patentblatt 88/32**

(84) Benannte Vertragsstaaten:
**AT CH FR GB IT LI LU NL SE**

(56) Entgegenhaltungen:
**EP-A-0 021 207**
**US-A-4 256 759**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Walker, Gordon Northrop, Dr., Lake Trail West Mount Kemble Lake, Morristown New Jersey (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

In der EP-A-0021207 bzw. US-A-4,256,759 sind β-Oxo-α-phenyl-carbamoyl-pyrrolpropionitrile, welche in 1-Position des Pyrrolrings durch Niederalkyl oder Phenyl-niederalkyl substituiert sind, mit antiinflammatorischen, antiarthritischen und immunopotenzierenden Eigenschaften beschrieben worden.

Der vorliegenden Erfindung liegt die Aufgabe zugrunde, neue Verbindungen bereit zu stellen, die zur Behandlung von inflammatorischen Erkrankungen geeignet sind, wie z. B. rheumatoide Arthrose und insbesondere Osteoarthrose, welche auch analgetische Wirkung aufweisen.

Die Aufgabe wird erfindungsgemäß durch die Bereitstellung der neuen analgetisch, antiinflammatorisch, antiarthritisch und immunopotenzierend wirksamen in 1-Stellung des Pyrrolylrestes unsubstituierten β-Oxo-α-carbamoyl-pyrrolpropionitril-Verbindungen gelöst.

Die Erfindung betrifft α-(gegebenenfalls substituierte Phenyl-carbamoyl)-pyrrolpropionitrile, welche in 1-Stellung des Pyrrolringes unsubstituiert sind und die Formel I

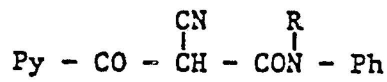

(I)

oder ihre tautomere Form aufweisen, worin Py einen 2- oder 3-Pyrrolyl-rest bedeutet, der in der 1-Stellung unsubstituiert ist und in einer oder mehreren der übrigbleibenden drei Stellungen gegebenenfalls durch Niederalkyl und/oder Carboxy oder niederes Carbalkoxy und/oder Halogen substituiert ist, R Wasserstoff oder Niederalkyl ist, und Ph Phenyl bedeutet, das unsubstituiert oder durch einen bis drei, gleiche oder verschiedene Substituenten der Gruppe Niederalkyl, Niederalkoxy, Niederalkylthio, Niederalkylsulfinyl, Niederalkylsulfonyl, Hydroxy, Halogen, Trifluomethyl, Nitro, Amino oder Niederalkanoylamino substituiert ist; ihre Salze, insbesondere ihre therapeutisch verwendbaren Salze; ihre Niederalkyl-enoläther oder Niederalkanoyl-enolester, wobei die mit "nieder" bezeichneten Reste höchstens 7 Kohlenstoffatome enthalten, Verfahren zu ihrer Herstellung, pharmazeutische Präparate enthaltend diese Verbindungen, sowie ihre therapeutische Verwendung.

Insbesondere betrifft die Erfindung Verbindungen der Formel I, oder ihre tautomeren Formen, worin Py einen in 1-Stellung unsubstituierten 2- oder 3-Pyrrolylrest bedeutet, der in den übrigbleibenden drei Stellungen einen oder mehrere Substituenten der Gruppe Niederalkyl und Halogen, eine Carboxy- oder eine niedere Carbalkoxygruppe, oder ein oder zwei Niederalkyl oder Halogen zusätzlich zu einer Carboxy- oder einer niederen Carbalkoxygruppe aufweisen kann; Ph für einen gegebenenfalls durch eine oder zwei gleiche oder verschiedene Substituenten der Gruppe Niederalkyl, Niederalkoxy, Niederalkylthio, Hydroxy, Halogen, Nitro, Amino und Niederalkanoylamino substituierten Phenylrest steht; ihre Salze, insbesondere ihre therapeutisch verwendbaren Salze; ihre Niederalkyl-enoläther oder Niederalkanoyl-enolester.

Bevorzugt sind Verbindungen der Formel I oder ihre tautomeren Formen, worin Py gegebenenfalls in einem oder mehreren der Stellungen 3, 4 und 5 durch eine bis drei Niederalkylgruppen oder durch eine oder zwei Niederalkylgruppen zusätzlich zu einer Carboxy- oder einer niederen Carbalkoxygruppe in 3 oder 4-Stellung, substituiertes 2-Pyrrolyl bedeutet; Ph für einen gegebenenfalls durch einen oder zwei, gleiche oder verschiedene Substituenten der Gruppe Niederalkyl, Halogen, Trifluormethyl, Niederalkylthio, Hydroxy oder Niederalkoxy substituierten Phenylrest steht; R Wasserstoff oder Niederalkyl bedeutet; ihre Salze, insbesondere ihre therapeutisch verwendbaren Salze; ihre Niederalkyl-enoläther oder Niederalkanoyl-enolester.

Besonders bevorzugt sind Verbindungen der Formel II

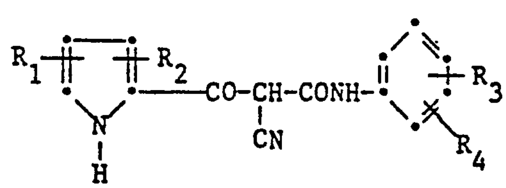

(II)

und ihre tautomeren Formen, worin jedes der Symbole $R_1$ und $R_2$ Wasserstoff oder Niederalkyl bedeutet, und jedes der Symbole $R_3$ und $R_4$ für Wasserstoff, Niederalkyl, Niederalkoxy, Hydroxy, Halogen oder Trifluormethyl steht; oder ihre Salze, insbesondere ihre therapeutisch verwendbaren Salze. Weitere bevorzugte Verbindungen sind diejenigen der Formel II, worin jedes der Symbole $R_1$ und $R_2$ Wasserstoff bedeutet; jedes der Symbole $R_3$ und $R_4$ für Wasserstoff, Alkyl mit höchstens 4 Kohlenstoffatomen, Fluor, Chlor oder Trifluormethyl steht; oder ihre Salze, insbesondere ihre therapeutisch verwendbaren Salze.

Sehr bevorzugt sind Verbindungen der Formel II, worin jedes der Symbole $R_1$ und $R_2$ Wasserstoff bedeutet; und jedes der Symbole $R_3$ und $R_4$ für Wasserstoff, Methyl, Methoxy, Fluor, Chlor oder Trifluormethyl steht; oder ihre Salze, insbesondere therapeutisch verwendbaren Salze, vorzugsweise ihre Natrium-, Kalium-, Calcium-, Triäthylammonium- oder Tris-(hydroxyäthyl)ammoniumsalze.

Bevorzugt sind insbesondere Verbindungen der Formel II, worin sich mindestens eines der Symbole $R_3$ und $R_4$ von Wasserstoff unterscheidet und sich in para-Stellung des Phenylringes befindet; und ihre Salze, insbesondere ihre therapeutisch verwendbaren Salze, vorzugsweise ihre Natrium-, Kalium-, Calcium-, Triäthylammonium- oder Tris-(hydroxyäthyl)ammoniumsalze.

Die hier verwendeten allgemeinen Definitionen haben im Rahmen der vorliegenden Erfindung die folgende Bedeutung.

Der Ausdruck "nieder" definiert in den oben und nachfolgend genannten organischen Resten und Verbindungen solche mit höchstens 7, vorzugsweise 4, insbesondere 1 oder 2 Kohlenstoffatomen.

Eine Niederalkylgruppe enthält vorzugsweise 1 - 4 Kohlenstoffatome und bedeutet zum Beispiel Äthyl, Propyl, Butyl oder vorzugsweise Methyl.

Eine Niederalkoxygruppe enthält vorzugsweise 1 - 4 Kohlenstoffatome und bedeutet zum Beispiel Äthoxy, Propoxy, Isopropoxy oder vorzugsweise Methoxy. Eine Niederalkylthiogruppe enthält vorzugsweise 1 - 4 Kohlenstoffatome und bedeutet vorzugsweise Methylthio oder Äthylthio. Eine Niederalkylsulfinylgruppe enthält vorzugsweise 1 - 4 Kohlenstoffatome und steht vorzugsweise für Methylsulfinyl oder Äthylsulfinyl. Eine Niederalkylsulfonylgruppe enthält vorzugsweise 1 - 4 Kohlenstoffatome und bedeutet vorzugsweise Methylsulfonyl oder Äthylsulfonyl.

Halogen ist vorzugsweise Chlor oder Fluor, kann aber ebenso Brom oder Jod sein.

Niederalkanoylamino bedeutet vorzugsweise Acetylamino oder Propionylamino.

Eine niedere Carbalkoxygruppe bedeutet vorzugsweise Carboäthoxy oder Carbomethoxy.

Die tautomeren Formen von Verbindungen der Formel I können mit der entsprechenden Enolstruktur der Formel Ia

```
             CN       R
             |        |
Py - C = C - C - N - Ph          (Ia)
     |       ||
     OH      O
```

dargestellt werden, worin Py, R und Ph die vorher für die Verbindungen der Formel I angegebene Bedeutung haben, und sie stehen mit diesen Verbindungen im Gleichgewicht.

Die Verbindungen der Formel I, welche mit ihren tautomeren Formen im Gleichgewicht stehen, haben Säurecharakter. Sie bilden als Derivate der enolischen tautomeren Struktur der Formel Ia, Niederalkyl-enoläther, Niederalkanoyl-enolester und Salze. Die Salze, welche mit therapeutisch verwendbaren Basen gebildet werden, sind z. B. von einem Alkalimetall, Erdalkalimetall, Kupfer- oder Zinkhydroxid, Ammoniak, Mono-, Di- oder Tri-(niederalkyl oder hydroxyniederalkyl)-aminen, monocyclischen Aminen oder Alkylendiaminen abgeleitet. Solche Salze sind z. B. Natrium-, Kalium-, Magnesium-, Ammonium-, Mono-, Di- oder Tri-(methyl, äthyl oder Hydroxyäthyl)-ammonium-, Pyrrolidinium-, Äthylendiammonium- oder Morpholiniumsalze oder ihre verschiedenen Hydrate.

Die Verbindungen der Erfindung zeigen wertvolle pharmakologische Eigenschaften, in erster Linie antiinflammatorische, analgetische (antinociceptive), antirheumatische, immunopotenzierende und antiarthritische Wirkungen. Diese können in in-vitro oder in-vivo Versuchen nachgewiesen werden. In den letzteren werden vorzugsweise Säugetiere, z. B. Ratten, Meerschweinchen oder Hunde als Testobjekte verwendet. Die Verbindungen der Erfindung können ihnen enteral, vorzugsweise oral, parenteral, z. B. subkutan oder intravenös, oder topisch, z. B. in Form von Lösungen in Wasser oder Öl, oder in Form von Stärke enthaltenden Suspensionen, verabreicht werden. Die verwendete Dosis kann in einem Bereich von ungefähr zwischen 0,1 und 100 mg/kg, vorzugsweise ungefähr 1 und 50 mg/kg/Tag, liegen. Die für die genannten Wirkungen gewählten Tests sind entweder klassische Versuchsmethoden, z. B. der Carrageenin-Pfotenödem- oder der Adjuvansarthritis-Test in der Ratte, der Synovitis-Versuch am Hund oder der Test, in welchem das durch ultraviolettes Licht hervorgerufene Erythem ausgewertet wird, oder moderne Testmethoden. Solche sind die neutrale Protease Hemmung [beschrieben in Arthritis Rheum. 17, 47 (1974)] oder die Hemmung der Leukozyten Chemotaxis [Ann. N.Y. Acad. Sci 256, 177 (1975)]. Weitere Methoden sind die Abnahme der neutrophilen Adherenz [Amer.J. Med. 61, 597 (1976)] oder die Hemmung der Prostaglandin-Synthetase, welcher Test in Biochem. 10, 2372 (1971) beschrieben ist; oder der Phenylquinon-writhing test.

Die immunopotenzierenden Wirkungen werden an BCG-immunisierten Tieren in-vitro oder in-vivo festgestellt.

Die Erhöhung der durch die Zellen vermittelten Immunität wird in-vitro durch Messung der erhöhten Chemotaxis der Monocyten wie folgt bestimmt:

Männliche Charles River-Ratten von einem Gewicht von 250 - 300 g werden durch intradermale Injektionen von 0,1 ml Bacillus Calmette Guerin-Vakzine (BCG) immunisiert. Nach einer Woche wird den Versuchstieren, zwecks Auslösung der Anreicherung von Makrophagen, eine Injektion von 10 ml steriler 2 %-iger Reis-Stärke-

Lösung intraperitoneal verabreicht. Am elften Tag nach der Immunisierung werden die Tiere getötet und peritoneale Makrophagen mit 20 ml Gey'scher gepufferter Salzlösung, welche Heparin enthält (25 Einheiten /ml) , gesammelt. Die Zellen werden 10 Minuten bei 1000 Umdrehungen /Minute zentrifugiert, mit weiteren 50 ml Gey'scher Lösung bei gleicher Geschwindigkeit und Zeit gewaschen und dann in Gey'scher Lösung, welche 0,1 % Human-Serumalbumin enthält, un eine Konzentration von 2 x $10^6$ Zellen/ml zu erreichen, suspendiert.

Mit den Test-Substanzen werden 1 x $10^{-2}$ Mol-Lösungen in Dimethylacetamid hergestellt. Nachfolgende Verdünnungen werden mit Gey'scher Lösung gemacht und diese schliesslich zu der obigen Zellen-Suspension gegeben, um geeignete Endkonzentrationen von $10^{-4}$, $10^{-5}$, $10^{-6}$ und $10^{-7}$ Mol zu erreichen. Nach dem Einbringen der Suspensionen in den oberen Teil von modifizierten Boyden-Chemotaxis-Kammern, verbleiben die genannten Test-Substanzen mit den Zellen zusammen. Als chemotaktisches Mittel wird ein Rattenserum, das mit dem Lipopolysaccharid von Escherichia coli aktiviert ist (Difco), (1/10 Verdünnung bei pH = 7,1), verwendet. Dieses Serum wird in die unteren Teile der genannten Kammern eingefüllt. Der die Zellen enthaltende Teil der Kammer ist von der chemotaktischen Lösung durch eine Zellulose-Filtermembran, welche Poren von 8 Mikron aufweist, getrennt.

Die Kammern werden in dreifacher Wiederholung aufgestellt und 5 Stunden bei 37°C inkubiert. Als Kontrolle der Zellenwanderung werden Zellensuspensionen, welche keine Test-Verbindung enthalten, eingesetzt. Nach der Inkubation werden die Filter entfernt, fixiert und mit Eisenhämatoxylin nach Weigert gefärbt. Vier Felder der unteren Oberfläche des Filters werden bei einer 320-fachen Vergrösserung mikroskopisch untersucht. Als Index für die chemotaktische Aktivität wird der Durchschnitt der Anzahl der in jenen vier Feldern gezählten Neutrophilen verwendet.

Die Erhöhung der durch die Zellen vermittelten Immunität wird in-vivo an der mit BCG-immunisierten arthritischen Ratten durch Messung der Verzögerung der Überempfindlichkeitsreaktion, im wesentlichen wie in Current Therapeutic Research 30, S 34 (1981) beschrieben, gemessen.

Männliche Charles River Ratten von einem Gewicht von 325 - 400 g werden sensibilisiert, indem man jedem Versuchstier in die rechte hintere Pfotensohle 250 µg Mycobacterium tuberculosis (Difco), das in Freund'schem Adjuvans (Freund's incomplete adjuvant) suspendiert ist, injiziert. Die Tiere werden am 18. Tag nach der Adjuvans-Injektion mit 0,1 ml BCG-Vakzine intradermal immunisiert. Die Wirksubstanz wird peroral in einer Maisstärke-Suspension verabreicht. Die Kontrolltiere werden lediglich mit der Maisstärke behandelt. Alle Tiere werden am 29. Tag an der Haut durch intradermale Verabreichung von 10 µg PPD (purified protein derivative) getestet, um Hautreaktionen hervorzurufen. Der Durchmesser des Erythems und die Erhärtungsreaktion werden 24 Stunden nach der Antigen-Behandlung gemessen. Eine Abnahme des Erythem-Durchmessers deutet auf die erhöhte Zellenimmunität hin.

Der Carrageenin-Photenödem-Test in Hinsicht auf die antiinflammatorische Wirkung wird an der Ratte wie folgt durchgeführt:

Eine Stunde nach der oralen Verabreichung der Wirksubstanz wird in die Sohle einer hinteren Pfote 0,1 ml Carrageenin (1 %-ig) injiziert. In bestimmten Zeiten wird dann die Differenz der Schwellung zwischen der behandelten und der intakten gegenseitigen Pfote durch Quecksilber-Verdrängung gemessen.

Der übliche Adjuvans-Arthritis-Test für die anti-arthritische Aktivität wird im wesentlichen wie in Proc. Soc. Exp. Biol. Med. 137, 506 (1971) durchgeführt..

Der Phenyl-p-benzoquinon - induzierte Writhing Test für die analgetische (antinociceptive) Wirkung wird wie in J. Pharmacol Exp. Therap. 125, 237 (1959) an Maus durchgeführt.

Als Beispiele der Erfindung seien das β-Oxo-α-(phenylcarbamoyl)-β-(2-pyrrolyl)-propionitril des Beispeils 1 und das β-Oxo-α-(4-chlor-phenyl)-β-(2-pyrrolyl)-propionitril des Beispiels 2 genannt, welche bei einer peroralen Dosis von 100 mg/kg, gegen die durch Carrageenin hervorgerufenen Ödem an der Ratte, 3 Stunden nach der Verabreichung, einen 46 bzw. 63 %-igen Schutz gewähren. Analog ergibt das β-Oxo-α-(2,4-difluorphenylcarbamoyl)-β-(2-pyrrolyl)-propionitril des Beispiels 3, bei einer Dosis von 25 mg/kg/p.o. einen 33 %-igen Schutz.

Weiter sind die Verbindungen der Erfindung, z. B. die vorher genannten Verbindungen der Beispiele 1, 2 und 3 in dem üblichen Adjuvans-Arthritis-Test an der Ratte bei einer Dosis von 25 mg/kg/p.o. aktiv und sie gewähren einen Schutz von 47 %, 58 % bzw. 55 %.

Auf die immunopotenzierende Wirkung von Verbindungen der Erfindung weist die Tatsache hin, dass z. B. die vorher genannten Verbindungen der Beispiele 1 und 2 im Haut-Test an der BCG immunisierten Adjuvans-Arthritis-Ratte bei einer Dosis von 25 mg/kg/p.o. eine signifikante Wirkung zeigen.

Die Verbindungen der Erfindung sind auch aktiv im Knorpel-Synovialgewebe-Co-Kultivationssysten, einem Modell für das Studium des Knorpel-Matrix-Abbaus. Daraus lässt sich wiederum ihre Wirkung in Osteoarthrose ableiten. Das Modell wird wie folgt aufgebaut:

Die Proteoglykan-Matrix des Nasenseptum-Knorpels des Rindes wird in-vitro durch $^{35}$S-Einbau in die Glykosaminoglykane markiert. Dazu werden die Knorpelscheibchen in sulfatfreiem Medium mit $^{35}$S-Natriumsulfat inkubiert. Danach werden die $^{35}$S-markierten Knorpelscheibchen mit Explantaten von normalem Synovialgewebe während 4 Tagen in "Multi-Well"-Gewebekulturplaten co-kultiviert. Am Ende dieser Inkubationszeit wird die Radioaktivität in einem 100 µ-Aliquot des Mediums gemessen. Darauf werden die Knorpelscheibchen hydrolysiert und die Radioaktivität darin bestimmt. Daraus lässt sich errechnen, welcher Teil der $^{35}$S-markierten Glykosaminoglykane während der Co-Kultur ins Medium abgegeben werden; durch Vergleich von behandelten und unbehandelten (= Kontroll) Kulturen lässt sich die prozentuale Hemmung der Matrix-Zerstörung errechnen.

Als Beispiele der Erfindung seien das β-Oxo-α-(phenylcarbamoyl)-β-(2-pyrrolyl)-propionitril des Beispiels 1, das β-Oxo-α-(4-chlorphenyl-carbamoyl)-β-(2-pyrolyl)-propionitril des Beispiels 2 und das β-Oxo-α-(2,4-difluorphenylcarbamoyl)-β-(2-pyrrolyl)-propionitril des Beispiels 3, welche den Knorpel-Matrix-Abbau in-vitro in einem Konzentrationsbereich von ungefähr $10^{-7}$M bis $10^{-5}$M hemmen.

Wegen ihrer vorhergenannten vorteilhaften Eigenschaften sind die Verbindungen der Erfindung als analgetische antiinflammatorische, antiarthritische und immunopotenzierende Mittel nützlich und sie sind insbesondere z. B. für die Behandlung von Schmerzzuständen verschiedener Genese und von inflammatorischen Krankheiten, z. B. rheumatoide Arthrose und Osteoarthrose in Säugern, Menschen inklusive, verwendbar.

Die Verbindungen der Erfindung werden nach an sich bekannten Methoden, z. B. dadurch hergestellt, dass man

a) Verbindungen der Formel III und IV

PY-$COCH_2$-CN (III) und Ph-N=C=O (IV)

kondensiert, und, wenn notwendig, eine erhaltene Verbindung durch Umsetzung mit einem reaktionsfähigen Ester von R-OH N-substituiert, wobei Py, Ph und R die vorher angegebenen Bedeutungen haben, oder

b) die Gruppe Z von einer Verbindung der Formel V

```
              CN    R
              |     |
Z - Py - CO - CH - CON - Ph        (V) ,
```

abspaltet, worin Py, R und Ph die vorher angegebenen Bedeutung haben und Z eine Schutzgruppe am Pyrrol-Stickstoffatom ist, oder

c) eine Verbindung der Formel VI

```
Py - COCH - COOH        (VI)
       |
       CN
```

oder ein reaktionsfähiges funktionelles Derivat davon, mit einem Amin der Formel R-HN-Ph (VII) kondensiert, worin Py, R und Ph die oben angegebenen Bedeutungen haben, oder

d) in einer Verbindung der Formel VIII

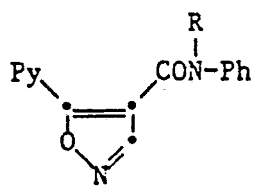

(VIII) ,

worin Py, R und Ph die vorher angegebenen Bedeutungen haben, den Ring öffnet, oder

e) eine Verbindung der Formel IX

Py-COOH (IX)

oder ein reaktionsfähiges funktionelles Derivat davon, mit einer Verbindung der Formel X

```
CH2 - CO - N - Ph        (X)
|          |
CN         R
```

kondensiert, worin Py, R und Ph die vorher angegebenen Bedeutungen haben, oder

f) Ammoniak oder ein Salz davon, mit einer Verbindung der Formel XI

```
               CN          R
               |           |
Y - CO - CH - CO - N - Ph          (XI)
```

umsetzt, worin R und Ph die vorher angegebenen Bedeutungen haben, und Y für unsubstituiertes oder substituiertes 2,5-Di-(niederalkoxy oder halogen)-2-tetrahydrofuranyl steht, und, wenn erwünscht, eine erhaltene Verbindung der Formel I in eine andere Verbindung der Erfindung umwandelt, und/oder, wenn erwünscht, ein erhaltenes Enol in ein Enol-niederalkyläther oder Enol-niederalkanoylester überführt, und/oder, wenn enwüscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

Die Kondensation gemäß dem Verfahren a), worin das Isocyanat der Formel IV mit dem Pyrroloylacetonitril der Formel III umgesetzt wird, kann gemäß dem U.S.-Patent 4,256,759 vorgenommen werden. Man arbeitet dabei in Abwesenheit oder in Gegenwart einer anorganischen oder organischen Base, z. B. Natriumhydrid oder Triäthylamin oder in Gegenwart oder Abwesenheit eines polaren Lösungsmittels, wie eines Äthers, z. B. Diäthyläther, Äthylenglykol-dimethyläther (Glyme) oder Tetrahydrofuran, und/oder eines Amids oder Sulfoxids, z. B. Dimethylformamid oder Dimethylsulfoxid; vorzugsweise in einem Temperaturbereich von 25° bis 100° C, insbesondere bei erhöhten Temperaturen, z. B. ungefähr 150°, wenn keine Base verwendet wird.

Das obergenannte Verfahren a) wird erfindungsgemäß wie folgt vorgenommen:

Das genannte Nitril wird mit einem geringen molaren Überschuss von wasserfreiem Triniederalkylamin, vorzugsweise Triäthylamin behandelt, und dann mit einem molaren Äquivalent des geeigneten Phenylisocyanats (Ph-N=C=O) oder einer Lösung davon in einem obengenannten polaren Lösungsmittel, z. B. Dimethylsulfoxid oder Glyme, versetzt. Das Reaktionsgemisch wird ungefähr 2 - 12 Stunden bei Raumtemperatur gerührt und sein Volumen unter Erwärmen bei nicht zu hohen Temperaturen vermindert. Der Rückstand wird mit einem Überschuss an verdünnter Säure, z. B. 0,1 - 0,3 normaler Chlorwasserstoffsäure, behandelt. Das erhaltene Rohprodukt wird extrahiert oder abgetrennt, mit Wasser gewaschen, getrocknet, trituriert und/oder aus geeigneten Lösungsmitteln umkristallisiert. Solche Lösungsmittel sind Niederalkanole, Niederalkanone, Diniederalkyläther und/oder Niederalkyl-niederalkanoate, z. B. Methanol, Aceton, Diäthyläther und/oder Essigsäureäthylester.

Die Ausgangsstoffe der Formeln III und IV sind bekannt oder sie können gemäß an sich bekannten Methoden hergestellt werden. So können z. B. Verbindungen der Formel III gemäß den in Ber. 113, 3675 (1980), Chem. Abstracts 29, 2164 und Ber. 55, 2390 (1922) beschriebenen Methoden erhalten werden.

Im Verfahren b) ist eine Schutzgruppe am Stickstoffatom des Pyrrolringes z. B. ein gegebenenfalls substituiertes Benzyl, gegebenenfalls substituiertes Carbobenzyloxy, Niederalkanoyl (z. B. Acetyl), Trifluoracetyl, Di-niederalkylamino (z. B. Dimethylamino), Tetrahydropyranyl, Niederalkoxymethyl (z B. Methoxymethyl) oder Niederalkoxycarbonyl (z. B. tert.-Butyloxycarbonyl).

Das Verfahren b), in welchem die Gruppe Z abgespalten wird, wird nach an sich bekannten Methoden durchgeführt. So wird z. B. 1) eine Hydrogenolyse vorgenommen, wenn Z gegebenenfalls substituiertes Benzyl oder Carbobenzyloxy bedeutet. Dazu verwendet man z. B Wasserstoff in Gegenwart eines Hydrierungskatalysators. Bedeutet Z gegebenenfalls substituiertes Carbobenzyloxy, Niederalkanoyl, Trifluoracetyl, Niederalkoxycarbonyl, Niederalkoxymethyl oder Tetrahydropyranyl, so wird die Abspaltung durch 2) Hydrolyse, vorzugsweise in Gegenwart z. B. einer Mineralsäure oder durch Ionenaustausch gemäß J. Am. Chem. Soc. 101, 6789 (1979) durchgeführt. Wenn Z für Dialkylamino steht, so kann diese Gruppe durch oxidative Spaltung, mit Chrom-II-acetat gemäß J. Org. Chem. 46, 3760 (1981) vorgenommen werden.

Die Ausgangsstoffe der Formel V können z. B. durch Kondensation einer Säure der Formel XII

```
                    CN
                    |
Z - Py - CO - CH - COOH        (XII)
```

oder eines reaktionsfähigen funktionellen Derivats davon, worin Z und Py die obige Bedeutung haben, mit einem Amin der Formel R-NH-Ph (VII) hergestellt werden.

Die Kondensation wird gemäß dem U.S.-Patent 4,256,759, vorzugsweise zwischen Raumtemperatur und 150° C, entweder mit äquivalenten Reagenzien-Mengen (wenn man einen reaktionsfähigen Ester verwendet), oder, zwecks Neutralisierung der gebildeten Säure, mit einem Amin-Überschuss, oder in Gegenwart einer anderen Base, z. B. eines tert.-Amins, wie Tri-niederalkylamin oder Pyridin (wenn ein Halogenid oder Anhydrid eingesetzt wird), vorgenommen. Das bei der Umsetzung mit den genannten Estern entstandene Niederalkanol wird vorzugsweise zusammen mit einem Verdünnungsmittel abdestilliert. Solche Mittel sind z. B. aromatische Kohlenwasserstoffe, z. B. Benzol, Toluol oder Xylol. Verwendet man eine freie Carbonsäure bei der Kondensation, so wird diese vorzugsweise in Gegenwart eines Kondensationsmittels, z. B. eines disubstituierten Carbodiimids wie Dicyclohexylcarbodiimid, 1,1'-Carbonyldiimidazol oder Diäthylphosphorocyanidat (Diäthyl-phosphoryl-cyanid) durchgeführt.

Die genannten Ausgangsstoffe der Formel V können auch gemäß den im U. S-Patent 4,256,759 beschriebenen Methoden hergestellt werden.

Die Kondensation des Verfahrens c) wird ananlog zu den für die Herstellung von Verbindungen der Formel V beschriebenen Methoden vorgenommen.

Die Ausgangsstoffe der Formel VI werden z. B. durch Umsetzung einer Verbindung der Formel III mit einem Kohlensäurederivat, z. B. Chlorameisensäure-äthylester, hergestellt Man erhält einen der Formel VI entsprechenden Äthylester, der in eine Verbindung der Formel VI oder in ein anderes reaktionsfähiges Derivat davon, nach an sich bekannten Methoden umgewandelt werden kann.

Die Ringöffnung-Reaktion des Verfahrens d) wird nach einer an sich bekannten, in J. Am. Chem. Soc. 35, 959 (1911) beschriebenen Methode, in Gegenwart einer starken anorganischen oder organischen Base, z. B. von Alkalimetallhydroxiden oder Tri-niederalkyl-aralkylammonium-hydroxiden, wie Trimethylbenzylammoniumhydroxid, durchgeführt.

Die Isoxazol-Ausgangsstoffe der Formel VIII werden auch nach an sich bekannten Methoden, z. B. wie in J. Am. Chem. Soc. 35, 959 (1913) beschrieben, hergestellt.

Die Konsation gemäss dem Verfahren e), wenn der Ausgangsstoff ein reaktionsfähiges funktionelles Derivat einer Verbindung der Formel IX ist, wird vorzugsweise in Gegenwart eines metallisierenden Mittels, z. B. eines Alkalimetalls, -metallalkoxids oder -hydrids, wie Natriumhydrid, Kalium-tert.-butoxid, Thallium-I-äthoxid, oder unter Phasentransfer-Bedingungen, in polaren Lösungsmitteln, z. B. 1,2-Dimethoxy-äthan, Dimethylformamid oder Dimethylsulfoxid, bei Temperaturen zwischen ungefähr 0° und 100°, vorzugsweise 25° bis 50°C, vorgenommen.

Reaktionsfähige funktionelle Derivate von Carbonsäuren der Formeln VI, IX und XII sind z. B. Anhydride, insbesondere gemischte Anhydride, Säurehalogenide, Säureazide, Niederalkylester oder aktivierte Ester. Gemischte Anhydride sind vorzugsweise solche der Pivalinsäure, oder eines Niederalkyl (Äthyl, Isobutyl)-hemiesters der Kohlensäure. Säurehalogenide sind z. B. Chloride oder Bromide. Aktivierte Ester sind z. B. Succinimido-, Phthalimido- oder 4-Nitrophenyl-ester. Niederalkylester sind z. B. Methyl- oder Äthylester.

Die Kondensation einer freien Carbonsäure der Formeln VI, IX und XII mit einer Verbindung der Formel VII oder X, gemäß den obigen Verfahren, kann in Gegenwart eines Kondensationsmittels, z. B. Diäthylphosphorocyanidat, in Gegenwart einer Base, z. B. Triäthylamin, in einem polaren Lösungsmittel, z. B. Dimethylformamid oder Methylenchlorid vorgenommen werden.

Die Umsetzung gemäß dem Verfahren f) betrifft vorzugsweise die Reaktion von Ammoniumacetat mit einer Verbindung der Formel XIa

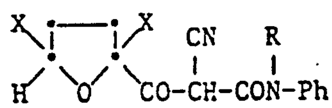

(XIa) ,

worin R und Ph die vorher angegebene Bedeutung haben, und X für Niederalkoxy oder Halogen, z. B. Methoxy, Äthoxy oder Chlor steht. Das Verfahren wird vorzugsweise in Eisessig bei erhöhter Temperatur, gemäß einer an sich bekannten Methodik, z. B. wie in Acta Chimica Scandinavica 6, 862 - 874 (1952) beschrieben, durchgeführt.

Die erhaltenen Verbindungen der Formel I können in an sich bekannter Weise ineinander übergeführt werden. So können z. B. erhaltene Enole, vorzugsweise mit Diazoalkanen, veräthert, oder z. B. mit Niederalkansäureanhydriden, verestert werden. Therapeutisch verwendbare Salze der genannten Enole können z. B. mit wässerigen Alkalimetallhydroxyden, vorzugsweise in Gegenwart eines Äthers oder Alkohols als Lösungsmittel, z. B. eines Niederalkanols, hergestellt werden. Aus den alkoholischen Lösungen können die Salze mit den genannten Äthern, z. B. Diäthyläther oder Tetrahydrofuran, ausgefällt werden. Man arbeitet bei mässigen Temperaturen, z. B, unter 100°. Erhaltene Salze können, wie oben erwähnt, durch Behandlung mit Säuren oder Basen in die freien Verbindungen umgewandelt werden. Diese oder andere Salze können auch zur Reinigung der erhaltenen Verbindungen verwendet werden. Infolge der engen Beziehung zwischen den neuen Verbindungen in freier Form und in Form ihrer Salze sind in vorausgegangenen und nachfolgend unter freien Verbindungen und Salzen sinn- und zweckgemäß gegebenenfalls auch die entsprechenden Salze bzw. freien Verbindungen zu verstehen.

Die Ausgangsstoffe sind bekannt, oder wenn neu, sie können nach an sich bekannten Verfahren, z. B. wie in der zitierten Literatur beschrieben oder wie in den Beispielen illustriert, hergestellt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensationsmitteln oder Neutralisationsmitteln, und/oder in einer inerten Atmosphäre, unter Kühlung, bei Zimmertemperatur oder bei erhöhten Temperaturen, bei normalem oder erhöhtem Druck durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder eines reaktionsfähigen Derivates, vorzugweise eines Alkalimetall-oder Trialkylammoniumsalzes der genannten Enole, verwendet wird. Beim Verfahren der vorliegenden Erfindung werden vorzugsweise solche Ausgangsstoffe verwendet, welche zu den eingangs als besonders wertvoll geschilderten Verbindungen führen.

Je nach der Wahl von Ausgangsstoffen und Methoden werden die neuen Verbindungen in der Form von Isomeren, Tautomeren oder ihren Gemischen, unter der Bedingungen, dass sie möglich sind, erhalten.

Die Verbindungen und ihre Salze können auch in Form ihrer Hydrate erhalten werden oder andere, für die Kristallisation verwendeten Lösungsmittel einschliessen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen, parentalen oder topischen Verabreichung eignen. Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schmiermitteln, z. B. Kieselerde, Talk, Stearinsäure oder Salze davon, wie Magnesium oder Calciumstearat, und/oder Polyäthylenglykol, aufweisen; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder das Natriumsalz davon, und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien oder topische Lotionen in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 1 % bis etwa 75 %, insbesondere von etwa 1 % bis etwa 50 %, des Aktivstoffes. Einzeldosen für Säuger mit einem Gewicht von ungefähr 50 - 70 kg können zwischen ungefähr 10 und 200 mg des aktiven Bestandteiles enthalten.

Die folgenden Beispiele dienen zur Illustration der Erfindung und sie dürfen nicht als ihre Einschränkung aufgefasst werden. Temperaturen werden in Celsiusgraden angegeben und die Angaben über Teile betreffen Gewichtsteile. Wenn nicht anders definiert, wird das Eindampfen von Lösungsmitteln unter vermindertem Druck, vorzugsweise zwischen ungefähr 15 und 100mm Hg (19,5 und 130 mbar) durchgeführt.

Beispiel 1: Eine Lösung von 3,1 g 2-Pyrroloylacetonitril in 25 ml Glyme (Äthylenglykoldimethyläther) und 2,7 g wasserfreiem Triäthylamin wird mit 3,0 g Phenylisocyanat behandelt. Nach einer mässig exothermischen Reaktion wird das Reaktionsgemisch über Nacht stehen gelassen. Der Grossteil des Lösungsmittels wird abgedampft und der Rückstand mit Wasser und 12 ml 10 %-iger Natriumhydroxidlösung behandelt. Die wässerige, alkalische Lösung wird mit Essigsäureäthylester gewaschen und mit 6-normaler Chlorwasserstoffsäure angesäuert. Das Produkt wird abgetrennt, mit Wasser gewaschen, getrocknet und aus Äthanol umkristallisiert. Man erhält das β-Oxo-α-(phenylcarbamoyl)-β-(2-pyrollyl)-propionitril, welches bei 207 - 209° schmilzt. Dies ist eine Verbindung der Formel II, worin $R_1$ bis $R_4$ Wasserstoff bedeuten.

Der Ausgangsstoff wird wie folgt hergestellt: Man leitet 20 Minuten in eine eisgekühlte Lösung von 30 g Pyrrol und 30 g Malonsäurenitril in 800 ml trockenem Äther trockenen Chlorwasserstoff ein. Man lässt das Reaktionsgemisch einige Stunden stehen und filtriert die Lösung. Man erhält 65 g eines orangebraunen, wasserlöslichen, festen Materials. Die Lösung von 50 g dieses Zwischenprodukts (Enamin-nitrilhydrochlorids) in 600 ml Wasser wird mit 400 ml Essigsäureäthylester überschichtet und 2,5 Stunden bei Raumtemperatur gerührt. Die organische Schicht wird abgetrennt, über Magnesiumsulfat getrocknet und eingedampft. Man erhält Kristalle, welche bei 77 - 79° schmelzen. Eine weitere Menge dieses Produkts wird durch Erhitzen der wässerigen Schicht auf dem Dampfbad für 30 Minuten und Extraktion mit Essigsäureäthylester erhalten. Umkristallisation aus Wasser ergibt das 2-Pyrroloylpropionitril F. 79 - 81°.

Beispiel 2: Eine Lösung von 6,7 g 2-Pyrroloylacetonitril in 25 ml Glyme und 5,5 g Triäthylamin wird mit einer Lösung von 8,6 g 4-Chlor-phenyl-isocyanat in 25 ml Glyme versetzt. Nach der exothermischen Reaktion wird das Gemisch über Nacht stehen gelassen Ein Teil des Lösungsmittels wird abgedampft und der gekühlte Rückstand wird in eine Lösung von 10 ml 6-normaler Chlorwasserstoffsäure in 250 ml Wasser gegossen. Man gibt etwas Methanol zum Gemisch und trennt das rohe Material ab. Es wird in verdünnter Natriumhydroxidlösung gelöst, die Lösung mit Aktivkohle behandelt, filtriert und mit 6-normaler Chlorwasserstoffsäure angesäuert. Das wieder ausgefällte Produkt wird abgetrennt, mit Wasser gewaschen und mit Methanol trituriert. F.219 - 221°. Nach Umkristallisation aus Methanol erhält man das β-Oxo-α-(4-chlorphenylcarbamoyl)-β-(2-pyrrolyl)-propionitril. F. 223 - 225°.

Beispiel 3: Durch Umsetzung von 2,4 g 2-Pyrroloylacetonitril mit 3,1 g 2,4-Difluorphenyl-isocyanat in Gegenwart von 2,0 g Triäthylamin in 25 ml Glyme und Isolierung analog zu den obigen Beispielen, dann Triturieren mit und Umkristallisation aus Äthanol, erhält man das β-Oxo-α-(2,4-difluorphenylcarbamoyl)-β-(2-pyrrolyl)-propinitril. F. 169 - 171°.

Beispiel 4: Umsetzung von 2,0 g 2-Pyrroloylacetonitril mit 3,0 g 3-(Trifluormethyl)-phenyl-isocyanat in Gegenwart von 1,8 g Triäthylamin in 25 ml Glyme analog zu den vorhergehenden Beispielen und Umkristallisation des Rohprodukts aus Methanol, ergibt das β-Oxo-α-(3-trifluormethyl-phenylcarbamoyl)-β-(2-pyrrolyl)-propionitril. F. 210 - 212°.

Beispiel 5: Umsetzung von 2,4 g 2-Pyrroloylacetonitril mit 2,5 g 4-Fluorphenyl-isocyanat in Gegenwart von 2,2

g Triäthylamin in 25 ml Glyme, analog zu den obigen Beispielen, nochmalige Ausfällung aus verdünnter Natriumhydroxidlösung mit Chlorwasserstoffsäure und Umkristallisation aus Methanol, ergibt das β-Oxo-α-(4-fluorphenyl-carbamoyl)-β-(2-pyrrolyl)-propionitril. F. 233 - 234°.

<u>Beispiel 6:</u> Umsetzung von 2,7 g 2-Pyrroloylpropionitril mit 3,8 g 3-Chlor-4-fluorphenyl-isocyanat in Gegenwart von 2,4 g Triäthylamin in 25 ml Glyme und Aufarbeitung analog zu den vorhergehenden Beispielen, ergibt nach Umkristallisation aus Essigsäureäthylester das β-Oxo-α-(3-chlor-4-fluorphenylcarbamoyl)-β-(2-pyrrolyl)-propionitril, welches unter Zersetzung bei 257 - 258° schmilzt.

<u>Beispiel 7:</u> Eine Lösung von 2,1 g 2-Pyrroloylacetonitril in 15 ml Glyme und 1,8 g Triäthylamin wird mit einer Lösung von 3 g 2,4-Di-chlorphenyl-isocyanat in 15 ml Glyme analog zu den obigen Beispielen behandelt. Nach einer schwach exothermischen Reaktion erhält man eine dicke Kristallsuspension. Man lässt sie über Nacht stehen, verdünnt sie mit trockenem Äther und filtriert sie. Es wird das Triäthylammoniumsalz des β-Oxo-α-(2,4-dichlorphenylcarbamoyl)-β-(2-pyrrolyl)-propionitril, F. 180 - 182° (Zersetzung) in der Form der Enolstruktur erhalten. Die Verbindung entspricht der Formel Ia, worin Py 2-Pyrrolyl bedeutet, R Wasserstoff ist und Ph für 2,4-Di-chlorphenyl steht.

Eine Lösung des obigen Triäthylammoniumsalzes in Methanol wird zu einer Lösung von 4 ml 6-normaler Chlorwasserstoffsäure in 250 ml Wasser gegeben. Das freie enolische Produkt wird abgetrennt, mit Wasser gewaschen und mit Methanol trituriert, F. 219 - 221°. Umkristallisation aus Essigsäureäthylester ergibt das β-Oxo-α-(2,4-di-chlorphenylcarbamoyl)-β-(2-pyrrolyl)-propionitril. F. 222 - 223°.

<u>Beispiel 8:</u> Ein Gemisch von 1,1 g β-Oxo-α-äthoxycarbonyl-β-(2-pyrrolyl)-propionitril, 1,5 g 4-Fluoranilin und 60 ml Xylol wird 4,5 Stunden unter Rückfluss gekocht. Man lässt das Gemisch über Nacht stehen und abkühlen, filtriert, dampft die Lösung ein und reinigt das Produkt. Man erhält das β-Oxo-α-(4-fluorphenylcarbamoyl)-β-(2-pyrrolyl)-propionitril des Beispiels 5.

Der Ausgangsstoff wird wie folgt hergestellt:

a) Eine Lösung von 2,22 g 2-Pyrrolcarbonsäure, 8,4 ml wasserfreies Triäthylamin und 2,1 ml Cyanessigsäure-äthylester in einer genügenden Menge Dimethylformamid wird gerührt, bei Raumtemperatur mit 2,9 g Diäthylphosphorocyanidat behandelt und 1,3 Stunden stehen gelassen. Die Lösung wird in einem Eisbad gekühlt, mit 50 ml Masser behandelt, filtriert und mit 6-normaler Chlorwasserstoffsäure angesäuert. Der erhaltene Niederschlag wird abgetrennt, mit Wasser gewaschen, getrocknet und aus Äther umkristallisiert. Man erhält das β-Oxo-α-äthoxycarbonyl-β-(2-pyrrolyl)-propionitril. F. 138 - 139°.

b) 2-Pyrroloylacetonitril wird in Gegenwart von Triäthylamin in Äthylenglykol-dimethyläther mit Chlorameisensäure-äthylester bei 50° über Nacht behandelt und das Produkt gereinigt. Man erhält das β-Oxo-α-äthoxycarbonyl-β-(2-pyrrolyl)-propionitril.

<u>Beispiel 9:</u> Gemäss den in den vorhergehenden Beispielen beschriebenen Verfahren können auch die folgenden Verbindungen der Formel I hergestellt werden:

| No. | Py | R | Ph |
|---|---|---|---|
| 9/1 | 2-pyrrolyl | H | 4-methoxyphenyl |
| 9/2 | 2-pyrrolyl | H | 4-methylthiophenyl |
| 9/3 | 2-pyrrolyl | H | 4-hydroxyphenyl |
| 9/4 | 2,5-dimethyl-3-pyrrolyl | H | 4-chlorophenyl |
| 9/5 | 3,5-dimethyl-2-pyrrolyl | H | 2,4-difluorophenyl |
| 9/6 | 3,5-dimethyl-4-carboethoxy-2-pyrrolyl | H | 4-chlorophenyl |
| 9/7 | 2-pyrrolyl | H | 4-trifluoromethylphenyl |
| 9/8 | 2-pyrrolyl | H | 4-tolyl |
| 9/9 | 2-pyrrolyl | $CH_3$ | phenyl |

Die Pyrrol-Ausgangsstoffe für die Beispiele 9/4, 9/5 und 9/6 sind in Ber. <u>113</u> (1980), Chem. Abstr. <u>29</u>, 2164 bzw. Ber. <u>55</u>, 2390 (1922) beschrieben.

<u>Beispiel 10:</u> Behandlung von β-Oxo-α-(2-Phenylcarbamoyl)-β-(2-pyrrolyl)-propionitril mit einer äquivalenten Menge konzentrierter wässeriger oder alkoholischer Lösung von Natrium-, Kalium- oder Calciumhydroxid oder -äthoxid und Eindampfen zur Trockene, ergibt das entsprechende Natrium-, Kalium- oder Calciumsalz der Verbindung der tautomeren Form der Formel Ia, worin Py 2-Pyrrolyl bedeutet, Ph Phenyl ist und R für Wasserstoff steht.

In analogen Weise werden mit äquivalenten Mengen von Triäthylamin oder Tri-(hydroxyäthyl)-amin das Triäthylammonium- und das Tris-(hydroxyäthyl)-ammoniumsalz hergestellt.

<u>Beispiel 11:</u> Man versetzt 500 ml ätherisches Diazomethan (welches ausgehend von 10,3 g N-Nitroso-N-methylharnstoff mit 35 ml 45 %-iger wässeriger Kaliumhydroxidlösung hergestellt und über Kaliumhydroxid-Tabletten getrocknet ist) mit 3,9 g β-Oxo-α-(phenylcarbamoyl)-β-(2-pyrrolyl)-propionitril. Nach dem Aufhören der Stickstoffentwicklung wird die Lösung filtriert und eingedampft. Nach Reinigung erhält man den entsprechenden Methyl-enoläther, d.h. das β-Methoxy-α-(phenylcarbamoyl)-β-(2-pyrrolyl)-acrylnitril.

Beispiel 12: Eine Lösung von 1,6 g Cyanacetanilid in 10 ml Dimethylformamid wird mit 3,4 g Kalium-tert.-butoxid versetzt. Die gerührte und gekühlte Suspension wird mit 1,1 g Pyrrol-2-carbonsäure in 6 ml Dimethylformamid und 1,4 ml Diäthylphosphorocyanidat (DEPC) behandelt. Die tiefrote Lösung wird in einem verschlossenem Gefäss eine halbe Stunde gehalten. Dann wird das Reaktionsgemisch mit 80 ml eiskaltem Wasser behandelt, filtriert, um das nicht reagierte Cyanacetanilid zu beseitigen, und mit 6-normaler Chlorwasserstoffsäure angesäuert. Der erhaltene Niederschlag wird abgetrennt, mit Wasser gewaschen und getrocknet. Das rohe Produkt wird in Essigsäureäthylester gelöst, die Lösung filtriert und zur trockene eingedampft.

Das erhaltene Produkt wird aus Äthanol-Äther umkristallisiert. Man erhält das β-Oxo-α-(phenylcarbamoyl)-β-(2-pyrrolyl)-propiontril. Das Produkt ist mit demjenigen des Beispiels 1 identisch.

Beispiel 13: Der Schutz im üblichen Adjuvans-Arthritis-Test, der im wesentlichen gemäß Proc. Soc. Exp. Biol. Med. 137, 506 (1971) durchgeführt wird, ist wie folgt:

| **Beispiel** | **% der Änderung im Vergleich zur Kontrolle** |
|---|---|
| 1 | - 47 % |
| 2 | - 58 % |
| 3 | - 55 % |
| 4 | - 59 % |
| 5 | - 59 % |
| 7 | - 42 % |

Beispiel 14: Herstellung von 1000 Kapseln mit einem Gehalt von je 25 mg der aktiven Substanz:

**Bestandteile:**

| | |
|---|---|
| β-Oxo-α-(4-fluorphenylcarbamoyl)-β-(2-pyrrolyl)-propionitril | 25,0 g |
| Milchzucker | 207,0 g |
| Maisstärke | 80,0 g |
| Magnesiumstearat | 3,0 g |

Verfahren:
Sämtliche pulverigen Bestandteile werden mit einem Sieb von 0,6 mm Maschenweite gesiebt. Dann wird der Wirkstoff zuerst mit Magnesiumstearat und darauffolgend mit Milchzucker und Stärke in einem geeigneten Mischer homogenisiert. Kapseln nr. 2 werden mit je 315 mg de Gemisches mit einer Füllmaschine gefüllt.

In analoger Weise werden Kapseln, welche 10 - 200 mg von anderen genannten Verbindungen enthalten, hergestellt.

Beispiel 15: Herstellung von 10.000 Tabletten mit einem Gehalt von je 100 mg der aktiven Substanz:

**Bestandteile:**

| | |
|---|---|
| β-Oxo-α-(2,4-difluorphenylcarbamoyl)-β-(2-pyrrolyl)-propionitril | 1000,00 g |
| Milchzucker | 2535 g |
| Maisstärke | 125 g |
| Polyäthylenglykol 6000 | 150 g |
| Talkpulver | 150 g |
| Magnesiumstearat | 40g |
| Gereinigtes Wasser | q.s. |

Verfahren:
Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 65 ml Wasser suspendiert und die Suspension zur siedenden Lösung von Polyäthylenglykol in 260 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm maschenweite getrieben und zu Tabletten, welche eine Bruchrille aufweisen, gepresst.

In analoger Weise werden Tabletten, welche eine andere, in vorhergehenden Beispielen illustrierte Verbindungen enthalten, hergestellt.

Beispiel 16: Die analgetische (antinociceptive) Wirkung wird an dem durch Phenyl-p-benzoquinon induziertem Writhing-Syndrom getestet (Hendershot und Forsaith: J. Pharmacol. Exp. Therap. 125, 237 (1959). Diese Methode wurde wie folgt modifiziert:

Mäusen, welche zuerst über Nacht gefastet haben, wird die Wirksubstanz, gelöst in 0,75 % Methylcellulose, in Dosen zwischen 1 bis 50 mg/kg p.o. 55 Minuten von der Induktion des Writhing-Syndroms verabreicht. Der Writhing Syndrom wird mit einer i.g. Injektion von 0,25 ml Suspension von Phenyl-p-benzoquinon (0,03 %) in Tragant (0,4 %) induziert.

5 Minuten danach beginnt die Zählung der Writhing-induzierten Reizbewegungen durch Beobachtung in einer Periode von 10 Minuten. Die antinociceptive $ED_{50}$ der getesteten Verbindung ist die Dosis, welche die Frequenz der induzierten Reizbewegungen auf 50 % im Vergleich mit Kontrolle reduziert.

**Patentansprüche** für die Vertragsstaaten CH FR GB ST LI LU NL SE

1. Verbindungen der allgemeinen Formel I

$$Py - CO - \underset{|}{\overset{CN}{}}CH - CO\overset{R}{\underset{|}{}}N - Ph \qquad (I)$$

oder ihre tautomeren Formen, worin Py einen 2- oder 3-Pyrrolylrest bedeutet, der in der 1-Stellung unsubstituiert ist und in einer oder mehreren der übrigbleibenden drei Stellungen gegebenenfalls durch Niederalkyl und/oder Carboxy oder niederes Carbalkoxy und/oder Halogen substituiert ist, R Wasserstoff oder Niederalkyl ist, und Ph Phenyl bedeutet, das unsubstituiert oder durch einen bis drei, gleiche oder verschiedene Substituenten der Gruppe Niederalkyl, Niederalkoxy, Niederalkylthio, Niederalkylsulfinyl, Niederalkylsulfonyl, Hydroxy, Halogen, Trifluormethyl, Nitro, Amino oder Niederalkanoylamino substituiert ist, wobei die mit "nieder" bezeichneten Reste höchstens 7 Kohlenstoffantome aufhalten; ihre Salze; ihre Niederalkyl-enoläther oder Niederalkanoyl-enolester.

2. Verbindungen der allgemeinen Formel II

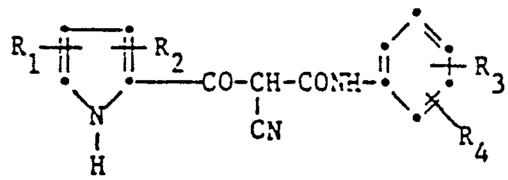 (II)

und ihre tautomeren Formen, worin jedes der Symbole $R_1$ und $R_2$ Wasserstoff oder Niederalkyl bedeutet, und jedes der Sole $R_3$ und $R_4$ für Wasserstoff, Niederalkyl, Niederalkoxy, Hydroxy, Halogen oder Trifluormethyl steht, wobei die mit "nieder" bezeichneten Reste höchstens 7 Kohlenstoffatome enthalten; oder ihre Salze.

3. β-Oxo-α-(phenylcarbamoyl)-β-(2-pyrrolyl)-propionitril.

4. β-Oxo-α-(4-chlorophenylcarbamoyl)-β-(2-pyrrolyl)-propionitril.

5. β-Oxo-α-(2,4-difluorophenylcarbamoyl)-β-(2-pyrrolyl)-propionitril.

6. β-Oxo-α-(3-trifluoromethylphenylcarbamoyl)-β-(2-pyrrolyl)-propionitril.

7. β-Oxo-α-(4-fluorophenylcarbamoyl)-β-(2-pyrrolyl)-propionitril.

8. β-Oxo-α-(3-chloro-4-fluorophenylcarbamoyl)-β-(2-pyrrolyl)-propionitril.

9. β-Oxo-α-(2,4-dichlorophenylcarbamoyl)-β-(2-pyrrolyl)-propionitril.

10. Pharmazeutische Präparate enthaltend Verbindungen gemäss einem der Ansprüche 1 bis 9 oder therapeutisch verwendbare Salze von solchen Verbindungen, zusammen mit einem pharmazeutischen Trägermaterial.

11. Verwendung von Verbindungen der Ansprüche 1 bis 9 zur Herstellung von pharmazeutischen Präparaten.

12. Die Verbindungen der Ansprüche 1 bis 9 zur Anwendung in einem Verfahren zur therapeutischen Behandlung des menschlichen oder tierischen Körpers.

13. Verwendung von Verbindungen der Ansprüche 1 bis 9 zur Herstellung von analgetisch wirksamen Arzneimitteln auf nicht-chemischen Wege.

14. Verfahren zur Herstellung von im Anspruch 1 definierten Verbindungen, dadurch gekennzeichnet, dass man

a) Verbindungen der Formel III und IV

$Py-COCH_2-CN$ (III) und $Ph-N=C=O$ (IV)

kondensiert, und, wenn notwendig, eine erhaltene Verbindung durch Umsetzung mit einem reaktionsfähigen Ester von R-OH N-substituiert, wobei Py, Ph und R die vorher angegebenen Bedeutungen haben, oder

b) die Gruppe Z von einer Verbindung der Formel V

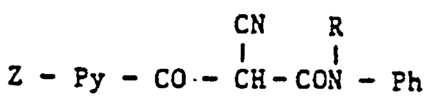 (V),

abspaltet, worin Py, R und Ph die vorher angegebenen Bedeutung haben und Z eine Schutzgruppe am Pyrrol-Stickstoffatom ist, oder

c) eine Verbindung der Formel VI

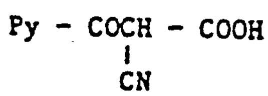 (VI)

oder ein reaktionsfähiges funktionelles Derivat davon, mit einem Amin der Formel R-NH-Ph (VII) kondensiert, worin Py, R und Ph die oben angegebenen Bedeutungen haben, oder

d) in einer Verbindung der Formel VIII

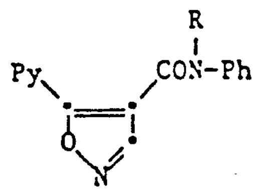

(VIII) ,

worin Py, R und Ph die vorher angegebenen Bedeutungen haben, den Ring öffnet, oder

e) eine Verbindung der Formel IX

Py - COOH (IX)

oder ein reaktionsfähiges funktionelles Derivat davon, mit einer Verbindung der Formel X

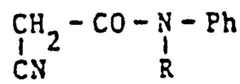

(X)

kondensiert, worin Py, R und Ph die vorher angegebenen Bedeutungen haben, oder

f) Ammoniak oder ein Salz davon, mit einer Verbindung der Formel XI

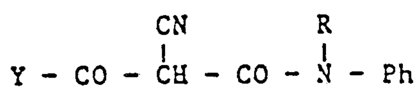

(XI)

umsetzt, worin R und Ph die vorher angegebenen Bedeutungen haben, und Y für unsubstituiertes oder substituiertes 2,5-Di-(niederalkoxy oder halogen)-2-tetrahydrofuranyl steht, und, wenn erwünscht, eine erhaltene Verbindung der Formel I in eine andere Verbindung der Erfindung umwandelt, und/oder, wenn erwünscht, ein erhaltenes Enol in ein Enol-niederalkyläther oder Enol-niederalkanoylester überführt, und/oder, wenn erwünscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

**Patentansprüche** für den Vertragsstaat AT

1. Verfahren zur Herstellung von Verbindungen der allgemeinen Formel

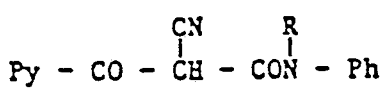

(I)

oder ihre tautomeren Formen, worin Py einen 2 oder 3-Pyrrolylrest bedeutet, der in der 1-Stellung unsubstituiert ist und in einer oder mehreren der übrigbleibenden drei Stellungen gegebenenfalls durch Niederalkyl und/oder Carboxy oder niederes Carbalkoxy und/oder Halogen substituiert ist, R Wasserstoff oder Niederalkyl ist, und Ph Phenyl bedeutet, das unsubstituiert oder durch einen bis drei, gleiche oder verschiedene Substituenten der Gruppe Niederalkyl, Niederalkoxy, Niederalkylthio, Niederalkylsulfinyl, Niederalkylsulfonyl, Hydroxy, Halogen, Trifluormethyl, Nitro, Amino oder Niederalkanoylamino substituiert ist wobei die mit "nieder" bezeichneten Reste höchstens 7 Kohlenstoffatome enthalten; ihren Salzen; ihren Niederalkyl-enoläther oder Niederalkanoyl-enolestern, dadurch gekennzeichnet, dass man

a) Verbindungen der Formel III und IV

Py-$COOH_2$-CN (III) und Ph-N=C=O (IV)

kondensiert, und, wenn notwendig, eine erhaltene Verbindung durch Umsetzung mit einem reaktionsfähigen Ester von R-OH N-substituiert, wobei Py, Ph und R die vorher angegebenen Bedeutungen haben, oder

b) die Gruppe Z von einer Verbindung der Formel V

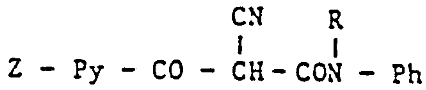 (V),

abspaltet, vorin Py, R und Ph die vorher angegebenen Bedeutung haben und Z eine Schutzgruppe am Pyrrol-Stickstoffatom ist, oder

c) eine Verbindung der Formel VI

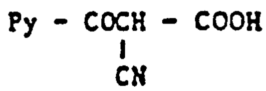 (VI)

oder ein reaktionsfähiges funktionelles Derivat davon, mit einem Amin der Formel R-NH-Ph (VII) kondensiert, worin Py, R und Ph die oben angegebenen Bedeutungen haben, oder

d) in einer Verbindung der Formel VIII

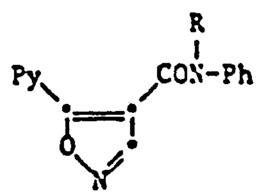 (VIII),

worin Py, R und Ph die vorher angegebenen Bedeutungen haben, den Ring öffnet, oder

e) eine Verbindung der Formel IX

Py - COOH (IX)

oder ein reaktionsfähiges funktionelles Derivat davon, mit einer Verbindung der Formel X

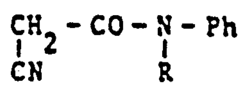 (X)

kondensiert, vorin Py, R und Ph die vorher angegebenen Bedeutungen haben, oder

f) Ammoniak oder ein Salz davon, mit einer Verbindung der Formel XI

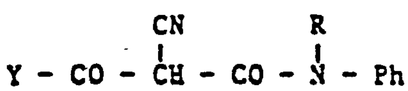 (XI)

umsetzt, worin R und Ph die vorher angegebenen Bedeutungen haben, und Y für unsubstituiertes oder substituiertes 2,5-Di-(niederalkoxy oder halogen)-2-tetrahydrofuranyl steht, und, wenn erwünscht, eine erhaltene Verbindung der Formel I in eine andere Verbindung der Erfindung umwandelt, und/oder, wenn erwünscht, ein erhaltenes Enol in ein Enol-niederalkyläther oder Enol-niederalkanoylester überführt, und/oder, wenn erwünscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

2. Verfahren nach Anspruch 1, dadurch gekennzeichnet, dass man Verbindungen der allgemeinen Formel II

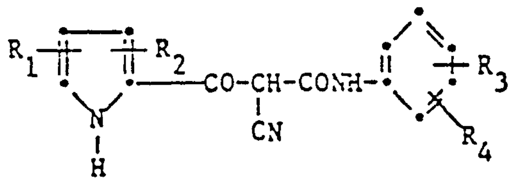

(II)

und ihre tautomeren Formen, worin jedes der Symbole $R_1$ und $R_2$ Wasserstoff oder Niederalkyl bedeutet, und jedes der Symbole $R_3$ und $R_4$ für Wasserstoff, Niederalkyl, Niederalkoxy, Hydroxy, Halogen oder Trifluormethyl steht, wobei mit "nieder" bezeichneten Reste höchstens 7 Kohlenstoffatome enthalten; oder ihre Salze herstellt.

3. Verfahren nach Anspruch 1, dadurch gekennzeichnet, dass man das β-Oxo-α-(phenylcarbamoyl)-β-(2-pyrrolyl)-propionitril oder seine Salze herstellt.

4. Verfahren nach Anspruch 1, dadurch gekennzeichnet, dass man das β-Oxo-α-(4-chlorphenylcarbamoyl)-β-(2-pyrrolyl)-propionitril oder seine Salze herstellt.

5. Verfahren nach Anspruch 1, dadurch gekennzeichnet, dass man das β-Oxo-α-(2,4-difluorphenylcarbamoyl)-β-(2-pyrrolyl)-propionitril oder seine Salze herstellt.

6. Verfahren nach Anspruch 1, dadurch gekennzeichnet, dass man das β-Oxo-α-(3-trifluormethylphenylcarbamoyl)-β-(2-pyrrolyl)-propionitril oder seine Salze herstellt.

7. Verfahren nach Anspruch 1, dadurch gekennzeichnet, dass man das β-Oxo-α-(4-fluorphenylcarbamoyl)-β-(2-pyrrolyl)-propionitril oder seine Salze herstellt.

8. Verfahren nach Anspruch 1, dadurch gekennzeichnet, dass man das β-Oxo-α-(3-chlor-4-fluorphenylcarbamoyl)-β-(2-pyrrolyl)-propionitril oder seine Salze herstellt.

9. Verfahren nach Anspruch 1, dadurch gekennzeichnet, dass man das β-Oxo-α-(2,4-dichlorphenylcarbamoyl)-β-(2-pyrrolyl)-propionitril oder seine Salze herstellt.

10. Verfahren zur Herstellung eines pharmazeutischen Präparates, gekennzeichnet durch die Verarbeitung eines erfindungsgemäßen Wirkstoffs nach einem der Ansprüche 1 bis 9 mit einem pharmazeutischen Trägermaterial.

**Claims** for the Contracting States CH FR GB IT LI LU NL SE

1. Compounds of the general formula I

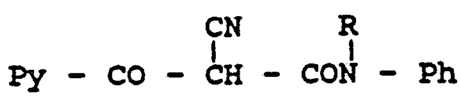

(I)

or tautomers thereof, wherein Py is 2- or 3-pyrrolyl unsubstituted at the 1-position and optionally substituted at one or more of the remaining three positions by lower alkyl and/or by carboxy or lower carbalkoxy and/or by halogen; R is hydrogen or lower alkyl; and Ph is phenyl unsubstituted or substituted by one to three identical or different substituents selected from the group lower alkyl, lower alkoxy, lower alkylthio, lower alkylsulfinyl, lower alkylsulfonyl, hydroxy, halogen, trifluoromethyl, nitro, amino and lower alkanoylamino, the radicals described as "lower" containing not more than 7 carbon atoms; the salts thereof; the lower alkyl enol ethers thereof; or the lower alkanoyl enol esters thereof.

2. Compounds of the general formula II

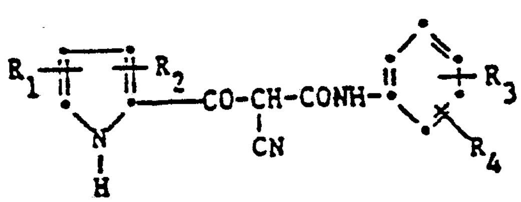

(II)

and tautomers thereof, wherein each of $R^1$ and $R^2$ is hydrogen or lower alkyl, and each of $R^3$ and $R^4$ is hydrogen, lower alkyl, lower alkoxy, hydroxy, halogen or trifluoromethyl, the radicals described as "lower" containing not more than 7 carbon atoms; or the salts thereof.

3. β-oxo-α-(phenylcarbamoyl)-β-(2-pyrrolyl)-propionitrile.

4. β-oxo-α-(4-chlorophenylcarbamoyl)-β-(2-pyrrolyl)-propionitrile.
5. β-oxoα-(2,4-difluorophenylcarbamoyl)-β-(2-pyrrolyl)-propionitrile.
6. β-oxo-α-(3-trifluoromethylphenylcarbamoyl)-β-(2-pyrrolyl)-propionitrile.
7. β-oxo-β-(4-fluorophenylcarbamoyl)-β-(2-pyrrolyl)-propionitrile.
8. β-oxo-α-(3-chloro-4-fluorophenylcarbamoyl)-β-(2-pyrrolyl)-propionitrile.
9. β-oxo-α-(2,4-dichlorophenylcarbamoyl)-β-(2-pyrrolyl) -propionitrile.
10. Pharmaceutical preparations containing compounds as claimed in any one of claims 1 to 9 or therapeutically acceptable salts of such compounds, together with a pharmaceutical carrier.
11. The use of compounds of claims 1 to 9 for the manufacture of pharmaceutical preparations.
12. The compounds of claims 1 to 9 for use in a method for the therapeutic treatment of the human or animal body.
13. The use of compounds of claims 1 to 9 for the manufacture of medicaments having analgesic action by a non-chemical route.
14. A process for the manufacture of compounds as defined in claim 1, characterised in that
a) compounds of the formulae III and IV

Py-$COCH_2$-CN (III) and Ph-N=C=O (IV)
are condensed, and, if necessary, a resulting compound is N-substituted by reaction with a reactive ester of R-OH, wherein Py, Ph and R are as defined above; or
b) the group Z is removed from a compound of the formula V

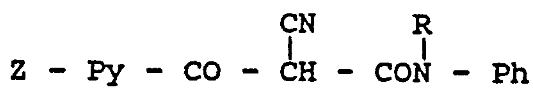

(V)

wherein Py, R and Ph are as defined above and Z is a protecting group on the pyrrole nitrogen; or
c) a compound of the formula VI

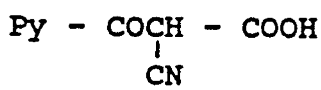

(VI)

or a reactive functional derivative thereof, is condensed with an amine of the formula R-NH-Ph (VII), wherein Py, R and Ph are as defined above; or
d) a compound of the formula VIII

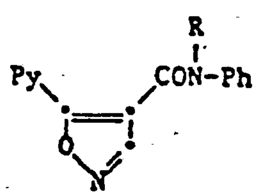

(VIII)

wherein Py, R and Ph are as defined above, is ring-opened; or
e) a compound of formula IX

Py - COOH (IX)
or a reactive functional derivative thereof, is condensed with a compound of formula X

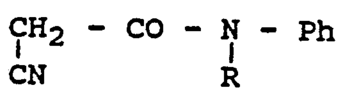

(X)

wherein Py, R and Ph are as defined above; or
f) ammonia or a salt thereof is reacted with a compound of formula XI

$$Y - CO - \overset{CN}{\overset{|}{C}}H - CO - \overset{R}{\overset{|}{N}} - Ph \qquad (XI)$$

wherein R and Ph are as defined above and Y represents unsubstituted or substituted 2,5-di-(lower alkoxy or halo)-2-tetrahydrofuranyl;

and, if desired, a resulting compound of formula I is converted into another compound of the invention, and/or, if desired, a resulting enol is converted into a lower alkyl enol ether or a lower alkanoyl enol ester, and/or, if desired, a resulting enol is converted into a salt with a base or a resulting enol salt is converted into the free enol or into another salt with a base, and/or, if desired, a mixture of isomers obtained is resolved into the single isomers.

**Claims** for the Contracting State AT

1. A process for the manufacture of compounds of the general formula

$$Py - CO - \overset{CN}{\overset{|}{C}}H - CO\overset{R}{\overset{|}{N}} - Ph \qquad (I)$$

or tautomers thereof, wherein Py is 2- or 3-pyrrolyl unsubstituted at the 1-position and optionally substituted at one or more of the remaining three positions by lower alkyl and/or by carboxy or lower carbalkoxy and/or by halogen; R is hydrogen or lower alkyl; and Ph is phenyl unsubstituted or substituted by one to three identical or different substituents selected from the group lower alkyl, lower alkoxy, lower alkylthio, lower alkylsulfinyl, lower alkylsulfonyl, hydroxy, halogen, trifluoromethyl, nitro, amino and lower alkanoylamino, the radicals described as "lower" containing not more than 7 carbon atoms; the salts thereof; the lower alkyl enol ethers thereof; or the lower alkanoyl enol esters thereof, characterised in that

a) compounds of the formulae III and IV

$Py\text{-}COCH_2\text{-}CH$ (III) and $Ph\text{-}H{=}C{=}O$ (IV)

are condensed, and, if necessary, a resulting compound is N-substituted by reaction with a reactive ester of ROH, wherein Py, Ph and R are as defined above; or

b) the group Z is removed from a compound of the formula V

$$Z - Py - CO - \overset{CN}{\overset{|}{C}}H - CO\overset{R}{\overset{|}{N}} - Ph \qquad (V)$$

wherein Py, R and Ph are as defined above and Z is a protecting group on the pyrrole nitrogen; or

c) a compound of the formula VI

$$Py - CO\underset{CN}{\underset{|}{C}}H - COOH \qquad (VI)$$

or a reactive functional derivative thereof, is condensed with an amine of the formula R-NH-Ph (VII), wherein Py, R and Ph are as defined above; or

d) a compound of the formula VIII

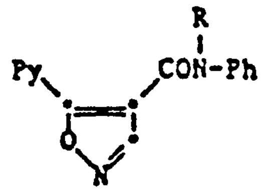

(VIII)

wherein Py, R and Ph are as defined above, is ring-opened; or

e) a compound of formula IX

Py - COOH (IX)

or a reactive functional derivative thereof, is condensed with a compound of formula X

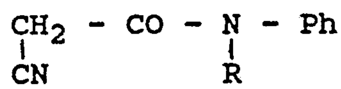

(X)

wherein Py, R and Ph are as defined above; or

f) ammonia or a salt thereof is reacted with a compound of formula XI

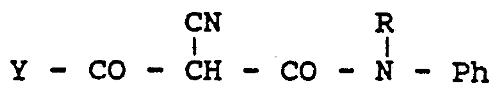

(XI)

wherein R and Ph are as defined above and Y represents unsubstituted or substituted 2,5-di-(lower alkoxy or halo)-2-tetrahydrofuranyl;

and, if desired, a resulting compound of formula I is converted into another compound of the invention, and/or, if desired, a resulting enol is converted into a lower alkyl enol ether or a lower alkanoyl enol ester, and/or, if desired, a resulting enol is converted into a salt with a base or a resulting enol salt is converted into the free enol or into another salt with a base, and/or, if desired, a mixture of isomers obtained is resolved into the single isomers.

2. A process according to claim 1, characterised in that compounds of the general formula II

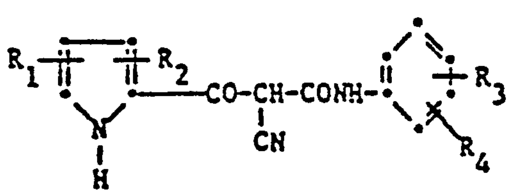

(II)

and tautomers thereof, wherein each of $R^1$ and $R^2$ is hydrogen or lower alkyl, and each of $R^3$ and $R^4$ is hydrogen, lower alkyl, lower alkoxy, hydroxy, halogen or trifluoromethyl, the radicals described as "lower" containing not more than 7 carbon atoms; or the salts thereof, are produced.

3. A process according to claim 1, characterised in that β-oxo-α-(phenylcarbamoyl)-β-(2-pyrrolyl)-propionitrile or a salt thereof is produced.

4. A process according to claim 1, characterised in that β-oxp-α-(4-chlorophenylcarbamoyl)-β-(2-pyrrolyl)-propionitrile or a salt thereof is produced.

5. A process according to claim 1, characterised in that β-oxo-α-(2,4-difluorophenylcarbamoyl)-β-(2-pyrrolyl)-propionitrile or a salt thereof is produced.

6. A process according to claim 1, characterised in that β-oxo-α-(3-trifluoromethylphenylcarbamoyl)-β-(2-pyrrolyl)-propionitrile or a salt thereof is produced.

7. A process according to claim 1, characterised in that β-oxo-α-(4-fluorophenylcarbamoyl)-β-(2-pyrrolyl)-propionitrile or a salt thereof is produced.

8. A process according to claim 1, characterised in that β-oxo-α-(3-chloro-4-fluorophenylcarbamoyl)-β-(2-pyrrolyl)-propionitrile or a salt thereof is produced.

9. A process according to claim 1, characterised in that β-oxo-α-(2,4-dichlorophenylcarbamoyl)-β-(2-pyrrolyl)-propionitrile or a salt thereof is produced.

10. A process for the manufacture of a pharmaceutical preparation, characterised in that an active ingredient

of the invention according to any one of claims 1 to 9 is processed with a pharmaceutical carrier.

**Revendications** pour les Etats contractants CH FR GB IT LI LU NL SE

1. Composés de formule générale I

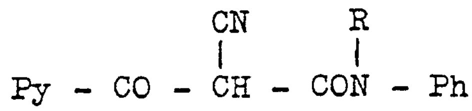

(I)

ou leurs formes tautomères, dans lesquels Py représente un groupe 2- ou 3-pyrrolyle non substitué en position 1 et substitué éventuellement dans une ou plusieurs des trois autres positions restantes par des groupes alkyle inférieurs et/ou carboxy ou carbalcoxy inférieurs et/ou des halogènes, R représente l'hydrogène ou un groupe alkyle inférieur et Ph représente un groupe phényle non substitué ou portant un à trois substituants identiques ou différents choisis parmi les groupes alkyle inférieurs, alcoxy inférieurs, alkylthio inférieurs, alkylsulfinyle inférieurs, alkysulfonyle inférieurs, hydroxy, les halogènes, les groupes trifluorométhyle, nitro, amino ou alcanoylamino inférieurs, les groupes qualifiés d'"inférieurs" contenant au maximum 7 atomes de carbone; leurs sels; leurs éthers alkyliques inférieurs d'énols ou esters alcanoyliques inférieurs d'énols.

2. Composés de formule générale II

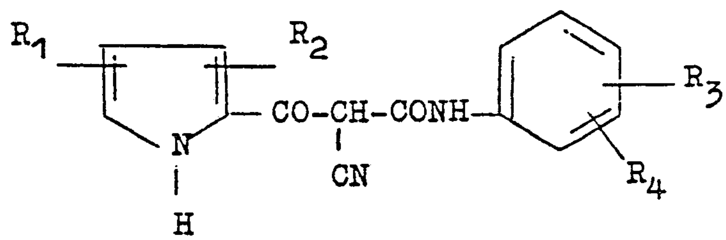

(II)

et leurs formes tautomères, dans lesquels chacun des symboles $R_1$ et $R_2$ représente l'hydrogène ou un groupe alkyle inférieur et chacun des symboles $R_3$ et $R_4$ représente l'hydrogène, un groupe alkyle inférieur, alcoxy inférieur, hydroxy, un halogène ou un groupe trifluorométhyle; les groupes qualifiés d'"inférieurs" contenant au maximum 7 atomes de carbone; ou leurs sels.

3. Le β-oxo-α-(phénylcarbamoyl)-β-(2-pyrrolyl)-propionitrile.

4. Le β-oxo-α-(4-chlorophénylcarbamoyl)-β-(2-pyrrolyl)-propionitrile.

5. Le β-oxo-α-(2,4-difluorophénylcarbamoyl)-β-(pyrrolyl)-propionitrile.

6. Le β-oxo-α-(3-trifluorométhtylphénylcarbamoyl)-β-(2-pyrrolyl)-propionitrile.

7. Le β-oxo-α-(4-fluorophénylcarbamoyl)-β-(2-pyrrolyl)-propionitrile.

8. Le β-oxo-α-(3-chloro-4-fluorophénylcarbamoyl)-β-(2-pyrrolyl)-propionitrile.

9. Le β-oxo-α-(2,4-dichlorophénylcarbamoyl)-β-(2-pyrrolyl)-propionitrile.

10. Compositions pharmaceutiques contenant des composés selon l'une des revendications 1 à 9 ou des sels utilisables en thérapeutique de tels composés, avec un véhicule pharmaceutique.

11. Utilisation des composés des revendications 1 à 9 pour la préparation de compositions pharmaceutiques.

12. Les composés des des revendications 1 à 9, pour l'utilisation dans un procédé pour le traitement thérapeutique de l'organisme humain ou animal.

13. Utilisation des composés des revendications 1 à 9 pour la préparation par voie non chimique de médicaments possédant une activité analgésique.

14. Procédé de préparation des composés définis dans la revendication 1, caractérisé en ce que:

a) on condense des composés de formules III et IV

$Py\text{-}COCH_2\text{-}CN$ (III) et $Ph\text{-}N{=}CO$ (IV)

et, lorsque c'est nécessaire, on substitue à l'azote un composé obtenu en le faisant réagir avec un ester réactif de R-OH, Py, Ph et R ayant les significations indiquées ci-dessus, ou bien

b) on élimine le groupe Z d'un composé de formule V

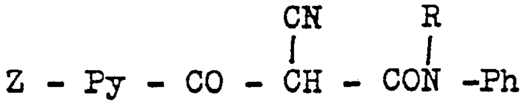

(V),

dans laquelle Py, R et Ph ont les significations indiquées ci-dessus et Z représente un groupe protecteur sur l'atome d'azote pyrrolique, ou bien

c) on condense un composé de formule VI

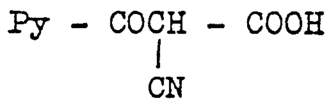

(VI)

ou un dérivé fonctionnel réactif d'un tel composé, avec une amine de formule R-NH-Ph (VII), Py, R et Ph ayant les significations indiquées ci-dessus, ou bien

d) on ouvre le cycle d'un composé de formule VIII

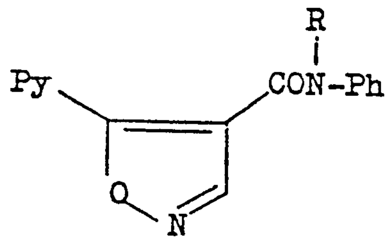

(VIII)

dans laquelle Py, R et Ph ont les significations indiquées ci-dessus, ou bien

e) on condense un composé de formule IX

Py - COOH (IX)

ou un dérivé fonctionnel réactif d'un tel composé, avec un composé de formule X

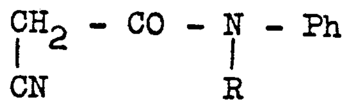

(X)

Py, R et Ph ayant les significations indiquées ci-dessus, ou bien

f) on fait réagir l'ammoniac ou un sel de l'ammoniac avec un composé de formule XI

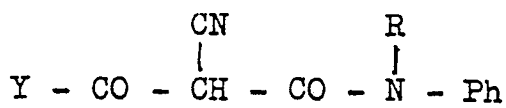

(XI)

dans laquelle R et Ph ont les significations indiquées ci-dessus et Y représente un groupe 2,5-di-(alcoxy inférieur ou halogéno)-2-tétrahydrofurannyle non substitué ou substitué et, si on le désire, on convertit un composé obtenu répondant à la formule I en un autre composé de l'invention et/ou, si on le désire, on convertit un énol obtenu en un éther alkylique inférieur d'énol ou un ester alcanoylique inférieur d'énol et/ou, si on le désire, on convertit un énol obtenu en un sel d'une base ou un sel d'énol obtenu en l'énol libre ou en un autre sel de base et/ou, si on le désire, on sépare un mélange d'isomères obtenu en les isomères individuels.

**Revendications** pour l'Etat contractant AT

1. Procédé de préparation des composés de formule générale

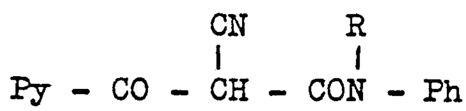

(I)

ou de leurs formes tautomères, dans lesquels Py représente un groupe 2- ou 3-pyrrolyle non substitué en position 1 et le cas échéant substitué dans une ou plusieurs des trois autres positions restantes par des groupes alkyle inférieurs et/ou carboxy ou carbalcoxy inférieurs et/ou des halogènes, R représente l'hydrogène ou un groupe alkyle inférieur et Ph un groupe phényle non substitué ou portant un à trois substituants identiques ou différents choisis parmi les groupes alkyle inférieurs, alcoxy inférieurs, alkylthio inférieurs, alkylsulfinyle inférieurs, alkylsulfonyle inférieurs, hydroxy, les halogènes, les groupes trifluorométhyle, nitro, amino ou alcanoylamino inférieurs, les groupes qualifiés d'"inférieurs" contenant au maximum 7 atomes de carbone; de leurs sels; de leurs éthers alkyliques inférieurs d'énols ou de leurs esters

alcanoyliques inférieurs d'énols, caractérisé en ce que

a) on condense des composés de formules III et IV

Py-$COCH_2$-CN (III) et Ph-N=C=O (IV)

et, lorsque c'est nécessaire, on substitue à l'azote un composé obtenu en le faisant réagir avec un ester réactif de R-OH, Py, Ph et R ayant les significations indiquées ci-dessus, ou bien

b) on élimine le groupe Z d'un composé de formule V

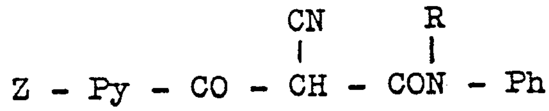
(V)

dans laquelle Py, R et Ph ont les significations indiquées ci-dessus et Z représente un groupe protecteur sur l'atome d'azote pyrrolique, ou bien

c) on condense un composé de formule VI

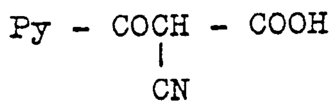
(VI)

ou un dérivé fonctionnel réactif d'un tel composé, avec une amine de formule R-NH-Ph (VII), Py, R et Ph ayant les significatons indiquées ci-dessus, ou bien

d) on ouvre le cycle d'un composé de formule VIII

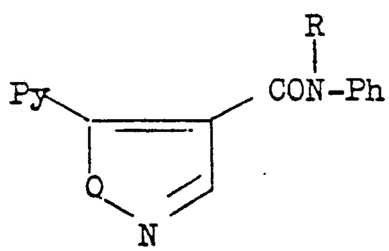
(VIII)

dans laquelle Py, R et Ph ont les significations indiquées ci-dessus, ou bien

e) on condense un composé de formule IX

Py - COOH (IX)

ou un dérivé fonctionnel réactif d'un tel composé, avec un composé de formule X

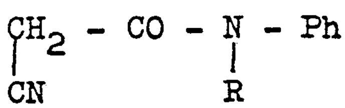
(X)

Py, R et Ph ayant les significations indiquées ci-dessus, ou bien

f) on fait réagir l'ammoniac ou un sel de l'ammoniac avec un composé de formule XI

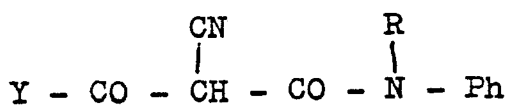
(XI)

dans laquelle R et Ph ont les significations indiquées ci-dessus et Y représente un groupe 2,5-di-(alcoxy inférieur ou halogéno)-2-tétrahydrofurannyle non substitué ou substitué et, si on le désire, on convertit un composé obtenu répondant à la formule I en un autre composé de l'invention et/ou, si on le désire, on convertit un énol obtenu en un éther alkylique inférieur d'énol ou un ester alcanoylique inférieur d'énol et/ou, si on le désire, on convertit un énol obtenu en un sel d'une base ou un sel d'énol obtenu en l'énol libre ou en un autre sel de base et/ou, si on le désire, on sépare un mélange d'isomères obtenu en les isomères individuels.

2. Procédé selon la revendication 1, caractérisé en ce que l'on prépare des composés de formule générale II

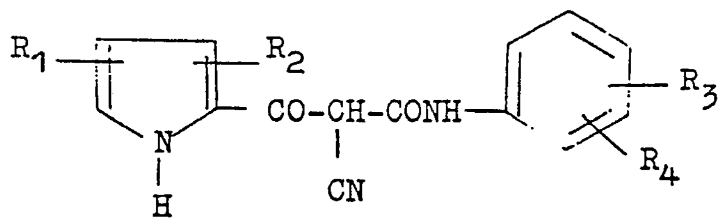

(II)

et leurs formes tautomères, dans lesquels chacun des symboles $R_1$ et $R_2$ représente l'hydrogène ou un groupe alkyle inférieur et chacun des symboles $R_3$ et $R_4$ représente l'hydrogène, un groupe alkyle inférieur, alcoxy inférieur, hydroxy, un halogène ou un groupe trifluorométhyle, les groupes qualifiés d'"inférieurs" contenant au maximum 7 atomes de carbone; ou leurs sels.

3. Procédé selon la revendication 1, caractérisé en ce que l'on prépare le β-oxo-α-(phénylcarbamoyl)-β-(2-pyrrolyl)-propionitrile ou ses sels.

4. Procédé selon la revendication 1, caractérisé en ce que l'on prépare le β-oxo-α-(4-chloro-phénylcarbamoyl)-β-(2-pyrrolyl)-propionitrile ou ses sels.

5- Procédé selon la revendication 1, caractérisé en ce que l'on prépare le β-oxo-α-(2,4-difluoro-phénylcarbamoyl-β-(2-pyrrolyl)-propionitrile ou ses sels.

6. Procédé selon la revendication 1, caractérisé en ce que l'on prépare le β-oxo-α-(3-trifluoro-méthylphénylcarbamoyl)-β-(2-pyrrolyl)-propionitrile ou ses sels.

7. Procédé selon la revendication 1, caractérisé en ce que l'on prépare le β-oxo-α-(4-fluoro-phénylcarbamoyl)-β-(2-pyrrolyl)-propionitrile ou ses sels.

8. Procédé selon la revendication 1, caractérisé en ce que l'on prépare le β-oxo-α-(3-chloro-4-fluorophénylcabamoyl)-β-(2-pyrrolyl)-propionitrile ou ses sels.

9. Procédé selon la revendication 1, caractérisé en ce que l'on prépare le β-oxo-α-(2,4-dichloro-phénylcarbamoyl)-β-(2-pyrrolyl)-propionitrile ou ses sels.

10. Procédé de préparation d'une composition pharmaceutique caractérisé en ce que l'on combine une substance active selon l'invention, selon l'une des revendications 1 à 9, avec un véhicule pharmaceutique.